(19) Europäisches Patentamt
European Patent Office
Office européen des brevets

(11) Veröffentlichungsnummer: **0 219 058 B2**

(12) # NEUE EUROPÄISCHE PATENTSCHRIFT

(45) Veröffentlichungstag der neue Patentschrift: **12.10.94**

(51) Int. Cl.5: **A61K 6/08**, A61L 25/00, A61L 27/00, C08F 2/44

(21) Anmeldenummer: **86113967.3**

(22) Anmeldetag: **08.10.86**

Die Akte enthält technische Angaben, die nach dem Eingang der Anmeldung eingereicht wurden und die nicht in dieser Patentschrift enthalten sind.

(54) **Polymerisierbare Zementmischungen.**

(30) Priorität: **09.10.85 DE 3536076**

(43) Veröffentlichungstag der Anmeldung: **22.04.87 Patentblatt 87/17**

(45) Bekanntmachung des Hinweises auf die Patenterteilung: **26.06.91 Patentblatt 91/26**

(45) Bekanntmachung des Hinweises auf die Entsheidung über den Einspruch: **12.10.94 Patentblatt 94/41**

(84) Benannte Vertragsstaaten: **CH DE FR GB IT LI NL SE**

(56) Entgegenhaltungen:
**EP-A- 0 059 649**
**EP-A- 0 127 758**
**EP-A- 0 132 318**
**EP-B- 0 036 272**
**WO-A-82/01990**
**CH-A- 588 863**
**DE-A- 1 937 871**
**DE-B- 2 711 234**
**DE-B- 2 724 814**
**DE-C- 2 061 513**
**DE-C- 2 934 380**
**FR-A- 2 363 613**
**GB-A- 2 094 326**
**US-A- 4 542 167**
**US-A- 4 544 359**
**US-A- 4 886 843**

**E.C. Combe: "Zahnärztliche Werkstoffe", Carl Hanser Verlag München, Wien, Seiten 134 - 136 (1984)**

(73) Patentinhaber: **Ernst Mühlbauer KG**
**Fangdieckstrasse 61**
**D-22547 Hamburg (DE)**

(72) Erfinder: **Engelbrecht, Jürgen, Dr. Dipl.-Chem.**
**Petkumstrasse 8**
**D-2000 Hamburg 76 (DE)**

(74) Vertreter: **Wolff, Hans Joachim, Dr.jur. Dipl.-Chem. et al**
**BEIL, WOLFF & BEIL,**
**Rechtsanwälte,**
**Postfach 80 01 40**
**D-65901 Frankfurt am Main (DE)**

**EP 0 219 058 B2**

## Beschreibung

Die Erfindung betrifft polymerisierbare Zementmischungen, insbesondere zur Verwendung in der Zahnheilkunde und Medizin.

In der Zahnmedizin findet für verschiedene Verwendungszwecke eine Reihe von Zementen Anwendung, und zwar beispielsweise zum Befestigen von Kronen und Inlays sowie von orthodontischen Vorrichtungen, als Wurzelkanalfüllungsmaterial, als Unterfüllungsmaterial bei der Einbringung von dentalem Restaurationsmaterial zum Schutze der Zahnpulpe oder auch als Füllungsmaterial selbst.

In der Medizin dienen einige dieser Zemente als Knochenzemente. Zemente für dentale und medizinische Zwecke bestehen in der Regel aus einer Mischung von feinstteiligen Metalloxiden, Metallhydroxiden, Silikatzementschmelzen oder ionenfreisetzenden Gläsern, die mit einer Anrührflüssigkeit, die im wesentlichen Phosphorsäure oder Polycarbonsäuren oder auch Salicylsäurederivate enthält, zur Reaktion gebracht wird.

Zemente auf der Basis von Phosphorsäure und Silikatzementschmelzen (Silikatzemente) bzw. Metalloxiden (Phosphatzemente) zeigen zwar eine mehr (Silikatzement) oder weniger (Phosphatzement) mechanische Festigkeit, sind jedoch recht wenig pulpenverträglich, zu spröde und zu wasserlöslich.

Zemente auf der Basis von Polycarbonsäuren und Metalloxiden (Carboxylatzemente) bzw. ionenfreisetzenden Gläsern (Ionomerzemente) sind auch mehr (Ionomerzemente) oder weniger (Carboxylatzemente) mechanisch fest, sind jedoch recht gewebeverträglich und zeigen gute Haftung an der Zahnsubstanz. Jedoch auch sie sind zu spröde, leiden an der weiterhin noch zu hohen Auswaschbarkeit im wäßrigen Milieu, zeigen keine chemische Hartung an Kunststoffüllungsmaterialien und binden auch nicht mit diesen ab.

Zemente auf der Basis von Salicylaten mit Metalloxiden oder -hydroxiden, besonders mit Calciumhydroxid, werden als Pulpenüberkappungsmaterial und Wurzelkanalfüllungsmaterial eingesetzt (z.B. US-A-3 047 408). Zemente dieses Typs wirken durch ihren hohen pH-Wert als schützende Sperre gegen Säuren und andere toxische Substanzen, die in einigen Füllungsmaterialien enthalten sein können.

Außerdem bewirken sie die Bildung von Sekundärdentin. Die mechanische Festigkeit der ausgehärteten Produkte ist jedoch nicht sonderlich hoch und die relative Wasserlöslichkeit läßt das Material sich verhältnismäßig schnell auflösen.

Wesentlich für Zemente ist, daß ihre Aushärtung über Ionenreaktionen wie Neutralisations-, Salzbildungs-, Chelatbildungs- oder Kristallisationsreaktionen abläuft, und zwar in Gegenwart von Wasser.

Je nach Verwendungszweck haben sich Zemente in der zahnärztlichen oder medizinischen Praxis mehr oder minder gut bewährt.

Zemente finden hauptsächlich Anwendung als Unterfüllungsmaterialien, als Befestigungsmaterialien sowie in Ausnahmefällen bei Läsionen im gingivalen Bereich auch als Füllungsmaterial.

Die großen Nachteile der Zemente, ihre Auswaschbarkeit und geringe mechanische Belastbarkeit haben dazu geführt, daß Zemente als Füllungsmaterial weitgehend durch die dauerhafteren, höher zu belastenden, kantenfesteren, unlöslichen und kosmetisch vorteilhafteren, polymerisierbaren Kunststoffüllungsmaterialien, sogenannten "Composites", ersetzt sind.

Composites bestehen im wesentlichen aus einem polymerisierbaren Bindemittel, welches durch organische oder anorganische Füllstoffe verstärkt ist. Als polymerisierbares Bindemittel eignen sich Verbindungen mit olefinisch ungesättigten Gruppen, für dentale und medizinische Zwekke besonders die Ester der (Meth)-acrylsäure von einwertigen und mehrwertigen Alkoholen, gegebenenfalls im Gemisch mit anderen Vinylmonomeren.

Als anorganische Füllstoffe dienen feinteilige Mehle von Quarz, mikrofeiner Kieselsäure, Aluminiumoxid, Bariumgläsern und anderer mineralischer Teilchen, die an sich keine chemische Bindung mit den sie umgebenden polymerisierbaren Bindemitteln eingehen und darum meist mit einem polymerisierbaren Silan als Kopplungsmittel versehen sind, um einen guten Verbund mit den polymerisierbaren Bindemitteln zu geben. Wesentlich für Composites ist, daß ihre Aushärtung durch eine Polymerisation der olefinisch ungesättigten Gruppen des Bindemittels abläuft, und zwar als radikalische Reaktion, die keiner Gegenwart von Wasser bedarf.

Obwohl heutzutage hauptsächlich Composites (neben Amalgamen) als dentales Restaurationsmaterial verwendet werden, sind jedoch auch ihnen in ihrer Anwendung Grenzen gesetzt.

Wegen Gewebeirritationen oder aus Gründen der Toxizität sind für tiefergehende Zahnkavitäten, bei Restaurationen am Gingivalsaum und am Dentin die Anwendung der Composites eingeschränkt. Auch haften sie nicht an der Zahnsubstanz. In solchen Fällen werden meist Zemente auf der Basis von Polycarbonsäuren und Metalloxiden (Carboxylatzemente) oder ionenfreisetzenden Gläsern (Ionomer-Zemente) angewandt. Restaurationsmaterialien dieser Art sind weniger toxisch und zeigen gute Haftung an

Zahn- und Knochensubstanz.

Es hat nicht an Versuchen gefehlt, die mechanische Festigkeit, aber vor allen Dingen das Löslichkeitsverhalten, das Entmischungsverhalten und die Compatibilität von Zementen mit Composites zu verbessern.

Um die Wasserlöslichkeit zu mindern, wurden z.B. zu Calciumhydroxidzementen oder Carboxylatzementen Polymere wie Polystyrole, Polyvinylacetate oder Polyvinylbutyrale oder auch Paraffinöl, Leinöl, Kolophonium oder andere natürliche Harze zugesetzt.

Entmischungserscheinungen wurden durch oberflächenaktive Substanzen wie Zinkstearat oder Ethyltoluolsulfonamid gemindert.

Olefinische Doppelbindungen enthaltende Zusätze wie z.B. Ester des 5-Methoxyconiferins, sogenannte Syringate, verbunden mit der Zugabe von radikalisch reagierenden Katalysatoren, brachten eine etwas bessere mechanische Festigkeit, etwas geringere Löslichkeit sowie eine gewisse Bindungsfähigkeit zu Composites.

In der DE-B2 2 724 814 werden Vorprodukte für die Zubereitung von Knochenzementen beschrieben, die aus Mischungen aus polymerisierbaren klassischen Methylmonomeren, Polymethylmethacrylat und Bioglaskeramikpulvern bestehen und eine verbesserte mechanische Festigkeit zeigen. Sie entbehren jedoch typische Zementeigenschaften wie Zementabbindereaktion einer Säure mit der Glaskeramik und guter Adhäsion an der Knochensubstanz.

Aus der DE-A 1 937 871 sind Massen für das Füllen von Zähnen bekannt, die aus einem Füllstoff, Methylacrylat bzw. Methacrylsäure und einem Härtungsmittel bestehen, wobei jedoch keine Zementreaktion zwischen der Methacrylverbindung und dem Füllstoff stattfindet.

Aus der DE-B2 2 711 234 sind Klebemittel für Zähne und Zahnmaterial bekannt, die zwar polymerisierbare Säureverbindungen enthalten, jedoch keine reaktiven Füllstoffe aufweisen. Die dort erwähnten Füllstoffe, wie Aluminiumoxid oder Siliciumoxid lassen sich nicht mit den dort genannten Säuren zu einer Abbinde- bzw. Zementreaktion bringen.

Der Erfindung liegt die Aufgabe zugrunde, neue dentale Mischungen zu finden, die einerseits wesentliche Vorteilsmerkmale von Zementen auf Polycarbonsäure- oder Salicylat-Basis wie gute Haftung an Zahn- und Knochensubstanz und gute Gewebeverträglichkeit aufzeigen, andererseits jedoch Vorteilsmerkmale von Composites wie eine geringe Löslichkeit und größere mechanische Festigkeit aufweisen, mit Composites copolymerisiert werden können und keine ausgeprägten Entmischungserscheinungen zeigen.

Die Aufgabe wird erfindungsgemäß gelöst durch polymerisierbare Zementmischungen, enthaltend

a) polymerisierbare ungesättigte Monomere und/oder Oligomere und/oder Prepolymere, die Säuregruppen und/oder deren Salze und/oder reaktive Säurederivatgruppen enthalten,

b) feinteilige, reaktive Füllstoffe, die mit diesen Säuren oder Säurederivaten reagieren können nämlich Pulver von Phosphatzement (ZnO/MgO), Silikatzemente, Ionomerzementen oder von ionenaustauschenden Zeolithen, sowie

c) Härtungsmittel,

dadurch gekennzeichnet, daß die Komponenten a) und b) derart ausgewählt sind, daß die Säuregruppen oder Säurederivatgruppen gemäß a) mit den feinteilgen, reaktiven Füllstoffen gemäß b) ionisch zu einer Zementreaktion zu führen vermögen.

Überraschenderweise zeigte sich, daß man durch eine Kombination von einigen für die Haftung an der Zahnsubstanz entwickelten polymerisierbaren Harzmischungen mit solchen reaktiven Füllstoffen, die üblicherweise in Zementen als für die Abbindung wichtige Komponente enthalten sind, zu härtbaren Mischungen kommt, die sowohl radikalisch als auch über Ionenreaktionen aushärten. Dadurch kann eine große Palette von neuen Compositezementen erhalten werden, die verbesserte Eigenschaften und neue Möglichkeiten der Anwendung bieten.

Beispiele von polymerisierbaren ungesättigten Monomeren mit Säure- oder reaktiven Säurederivatgruppen, die auch als gute Haftvermittler auf oxidischen Materialien und der Zahnsubstanz bekannt sind, sind
ungesättigte organische Ester von Phosphor- oder Phosphonsäuren
(DE-B-27 11 234, DE-A-31 50 285);
ungesättigte organische Ester der Monofluorphosphorsäure
(US-A-3 997 504);
ungesättigte organische Ester von Säuren des Phosphors, die Chlor oder Brom direkt am Phosphor gebunden enthalten
(EP-A-0 058 483);
ungesättigte organische Ester der Phosphorsäure, die als cyclische Pyrophosphate (Anhydride) vorliegen
(DE-A-30 48 410);
4-Methacryloyloxyethyltrimellitsäure und deren Anhydrid (Takeyama, M. et al., I. Jap. Soc. f. Dent. App. a. Mat. 19, 179 (1978)) oder

Pyromellitsäure-bis-2-methacryloylethylester.

Beispiele von üblicherweise in Zementen als für die Abbindung wichtige Pulverkomponenten sind z.B. in DE-C-2061513, CH-A-588863, DE-A-2751 069, DE-A-2750326, US-A-4 250 277, EP-A-0023013, US-A-4 376835 beschrieben.

Die polymerisierbaren ungesättigten Mono-, Oligo- oder Prepolymeren der erfindungsgemäßen polymerisierbaren Zementmischungen können Alkenyl-, Alkenoxy-, Cycloalkenyl-, Aralkenyl- oder Alkenarylreste, vorteilhaft jedoch Acryl-, Methacryl-, Vinyl- oder Styryl-, und davon besonders vorteilhaft die Acryl- und Methacrylreste, die die polymerisierbaren Gruppen vieler Monomerer in Dentalmaterialien sind, tragen.

Als Säuregruppen können all jene Gruppen enthalten sein, die mit oxidischen, mineralischen, keramischen, glasartigen oder metallischen Füllstoffen reagieren können. Vorteilhafterweise werden diese Säuregruppen Carbonsäurereste, folgende Reste von Säuren des Phosphors

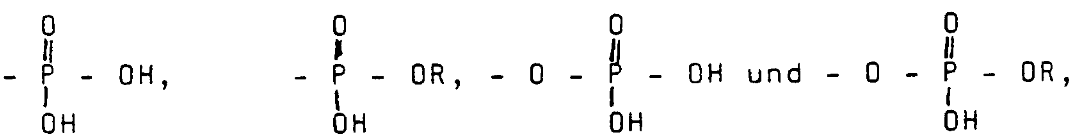

wobei R z.B. Alkenyl, Aryl oder Vinyl bedeutet, folgende Reste von Säuren des Schwefels

$-SO_2H$, $-SO_3H$ oder $-O-SO_3H$,

oder folgende Reste von Säuren des Bors

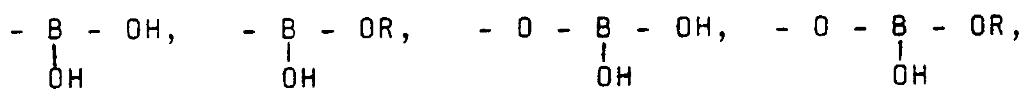

wobei R Alkenyl, Aryl oder Vinyl bedeutet, sein.

Kationensäurereste wie $-NR_2H^+$ oder $-PR_2H^+$ (R = H, Alkyl) sind ebenfalls geeignet.

Die reaktiven Säurederivatgruppen können Säurehalogenide, -anhydride, leicht zur Säure hydrolisierende Säureamide, -nitrile und -ester, wie z.B. Silyl- oder tert.-Butyl substituierte sein, sofern sie mit dem reaktiven Füllstoff zu Ionenaustausch-, Neutralisations-, Salzbildungs- oder Chelatbildungsreaktionen fähig sind.

Besonders bevorzugt sind Säuregruppen bzw. reaktive Säurederivate als Carboxylat-, Phosphat-, Phosphonat-, Sulfonat-oder Boratsäurereste bzw. als deren reaktiven Derivate.

Besonders eignen sich solche Verbindungen, die mindestens 2 polymerisierbare Gruppen oder mindestens 2 Säuregruppen bzw. Säurederivatgruppen enthalten.
Beispiele sind Phosphorsäureester von Glycerindimethacrylat oder 1-Methacryloxyethan-1,1-diphosphonsäure.

Ganz besonders bevorzugt werden solche Verbindungen, die mindestens 2 polymerisierbare Gruppen und mindestens 2 Säuregruppen enthalten wie die Chlor- oder Bromphosphorsäureester des Bisphenol-A-Glycidyldimethacrylates (Bis-GMA), welche durch Umsetzen von Bis-GMA mit Phosphorylchlorid leicht hergestellt werden können und wobei das Verhältnis von Phosphor zu Bis-GMA bei 2:1 liegt.

Beispiele von polymerisierbaren ungesättigten Oligomeren oder Prepolymeren mit Säure- oder Säurederivatgruppen sind solche Verbindungen, die an chemisch recht stabilen, molekularen Grundgerüsten sowohl polymerisierbare Gruppen als auch Säure- oder Säurederivategruppen gebunden enthalten.

Vorteilhafterweise enthalten die polymerisierbaren ungesättigten Oligomere oder Prepolymere 2 ungesättigte Gruppen und/oder 2 Säure- bzw. 2 reaktive Säurederivatgruppen, besonders vorteilhaft jedoch drei und mehr ungesättigte Gruppen und drei und mehr Säure- bzw. drei und mehr reaktive Säurederivatgruppen.

Solche Verbindungen sind sehr gute Haftvermittlerkomponenten auf oxidischem, mineralischem, keramischem, glasartigem, metallischem und biologischem Substrat, besonders der Zahnsubstanz, und eine besonders geeignete Komponente erfindungsgemäßer Zementmischungen.

Die molekularen Grundgerüste solcher Verbindungen können linearer, verzweigter oder zyklischer Natur sein.

Sie können Polymerisate von ethylenisch ungesättigten Monomeren, aber auch oligomere oder polymere Verbindungen wie z.B. Polyester, Polyamide, Polyether, Polyphosphazene, Polysaccharide usw. sein, soweit das Gerüst ausreichend hydrolysestabil ist, sie andererseits mit den gewünschten polymerisierbaren Gruppen versehen werden können, sie die gewünschten Säuregruppen tragen oder sie mit diesen versehen werden können.

Die gewünschten Gruppen können aufgepfropft werden, wenn das Grundgerüst eine Vielzahl von funktionellen Gruppen gebunden enthält, die eine solche Pfropfreaktion erlauben, wie z.B. Alkohol-, Halogen-, Säurehalogenid-, Amino-, Epoxid- oder Isocyanatgruppen.

Das bedeutet, daß oben genannte Grundgerüste ungeachtet dessen, aus welchen Bausteinen sie aufgebaut sind, z.B. in der Regel als Polyole, Polyhalogenide, Polysäurehalogenide, Polyamine Polyepoxide, Polyisocyanate, Polyanhydride in reiner oder gemischter Form zu den oligomeren oder polymeren Verbindungen der erfindungsgemäßen Mischungen führen können. Bevorzugte Grundgerüste sind Polymerisate aus ethylenisch ungesättigten Monomeren.

Die bevorzugten oligomeren oder prepolymeren Grundgerüstverbindungen die Grundlage zur Herstellung der bevorzugten oligomeren oder prepolymeren Verbindungen sind, können aus polymerisierbaren Monomeren durch eine geeignete Reaktionsführung zu Oligomeren oder Polymeren verschiedenen Polymerisationsgrades hergestellt werden.

Dabei ist einerseits eine Gruppe von Monomeren geeignet, die zu Homo-oligomerisaten oder Homopolymerisaten führt, andererseits eine Gruppe, die durch Kombination verschiedener Monomerer zu Cooligomerisaten oder Co-polymerisaten führt. Aus der Gruppe der Homopolymerisate sind z.B. Oligo-oder Polymerisate von ungesättigten Säuren, die als Säurechlorid eingesetzt werden, geeignet (A):

(A) 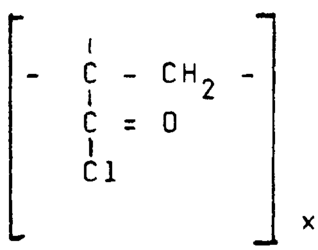

Sie können dann im ersten Schritt zu einem gewünschten Anteil mit z.B. Hydroxyethylmethacrylat umgesetzt und, falls die freie Säuregruppe gewünscht wird, darauf in einem zweiten Schritt der Rest der Säurechloride hydrolisiert werden. Die Verteilung der Gruppen erfolgt statistisch, z.B. (B):

(B) 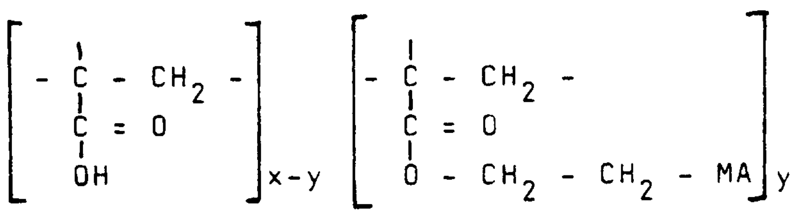

wobei MA einen Methacrylrest

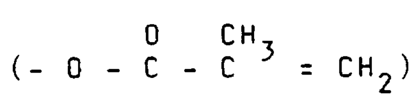

bedeuten soll.

Der zweite Schritt ( Hydrolyse) kann jedoch auch durch einen Alkoholyseschritt mit säuregruppenhaltigen Alkoholen wie 1-Hydroxyethan-1, 1-diphosphonsäure ersetzt werden, so daß Produkte wie (C)

(C) 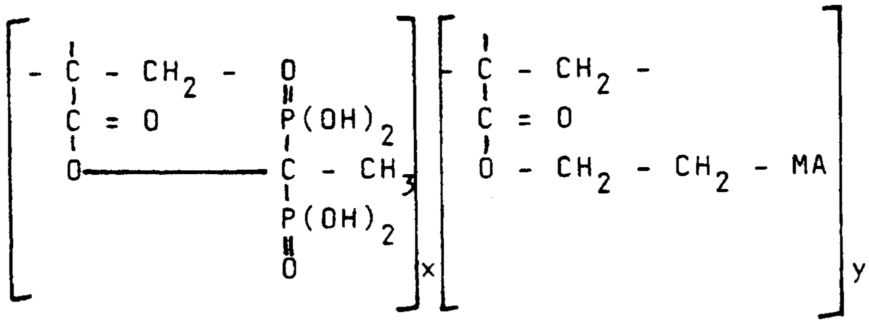

erhalten werden.

Ein weiteres gutes Grundgerüst für erfindungsgemäße Verbindungen sind Homopolymerisate aus ungesättigten Alkoholen (D). Ein Teil der Hydroxygruppen kann z.B. durch Veresterung mit einer ungesättigten Säure bzw. einem ungesättigten Säurechlorid mit polymerisierbaren Gruppen versehen werden; ein anderer Teil kann mit Säuren oder Säurechloriden wie z.B. Borsäure oder Phosphorylchlorid zu entsprechenden, erfindungsgemäßen Verbindungen (E, F) umgesetzt werden.

(D) 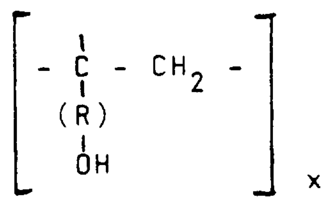

R ,R' = kein oder inerter Rest

(E) 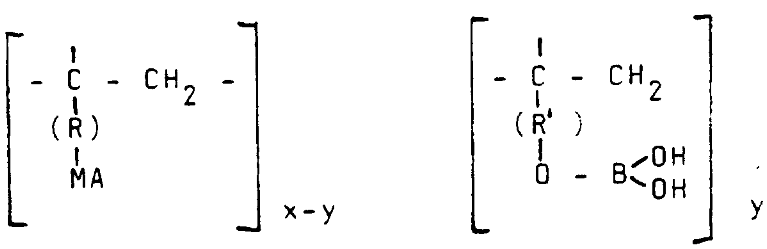

(F) 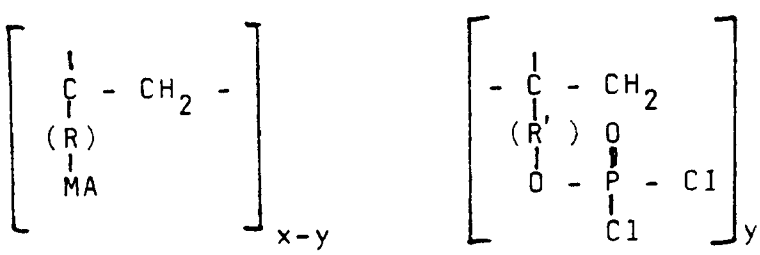

Liegt ein oligomeres oder polymeres Grundgerüst mit intramolekularen Anhydrid- oder intramolekularen Imidgruppen vor, können polymerisierbare Gruppen und Säuregruppen in einem Schritt angeknüpft werden.

Besonders bevorzugt sind Oligo- oder Polymerisate von Maleinsäureanhydrid:

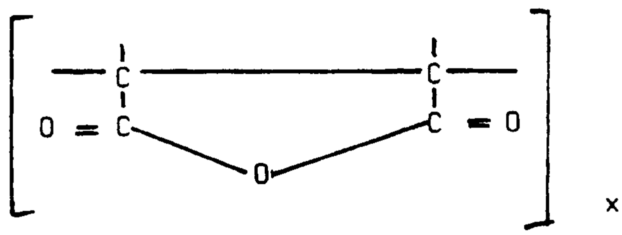

Dieses setzt sich z.B. mit einem Hydroxyalkylmethacrylat in Verhältnissen von 1:1 zu Produkten wie (G) um.

Bei Zugabe von weniger Hydroxymethacrylat und dafür zusätzlichem Hydroxysäurederivat kann leicht ein Produkt mit 2 verschiedenen Haftgruppen erhalten werden (H).

(G) 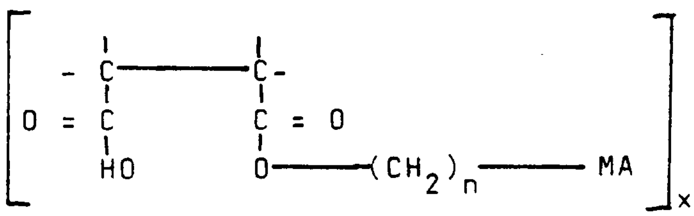 bzw.

(H) 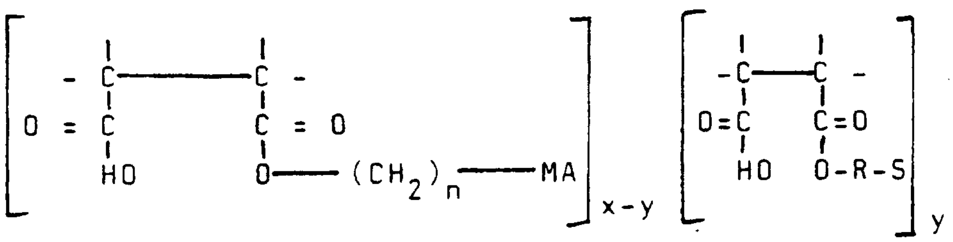

wobei S ganz allgemein der Säure- oder Säurederivatrest sein soll und R ein beliebiger Rest ist.

Bei einer weiteren Gruppe von bevorzugten Grundverbindungen, nämlich Co-oligomerisate oder Copolymerisate, werden Säure-oder säurederivathaltige Vinyl-, Styrol- oder (Meth)acrylmonomere wie Vinylphosphat, Vinylphosphonat, Methacrylsäureester von Phosphorsäuren oder Phosphonsäuren, Styrylverbindungen mit Säuregruppen des Phosphors, Bors und Schwefel, wie z.B. sulfoniertes Styrol mit ungesättigten Verbindungen wie Vinylchloracetat oder chlormethyliertem Styrol copolymerisiert so daß Verbindungen wie z.B. (I)

(I) 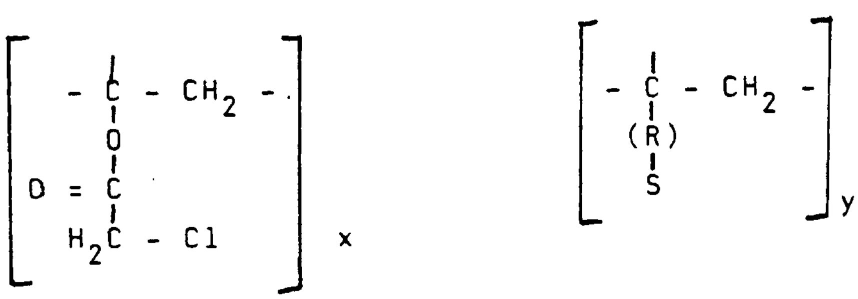

oder z.B. (K)

(K) 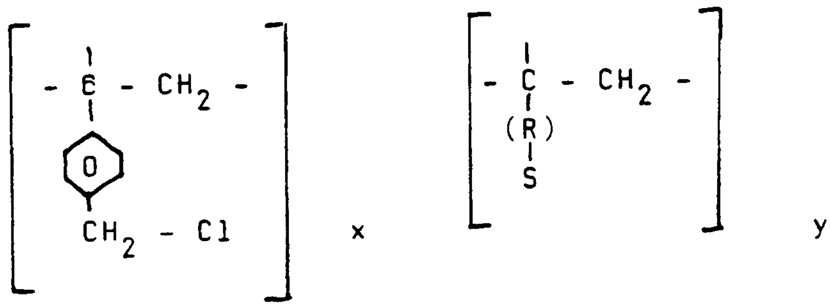

entstehen.

Verbindungen dieser Art können dann z.B. mit Natriummethacrylat in eine polymerisierbare Verbindung (L) überführt werden:

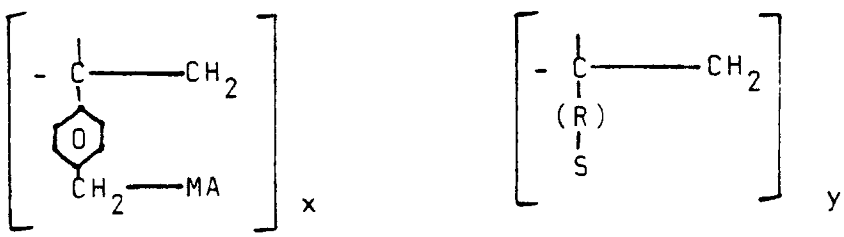

Auch Copolymerisate (M) von ungesättigten Alkoholen mit ungesättigten Säuren führen nach Reaktion mit Verbindungen wie z.B. Methacrylsäurechlorid zu Produkten (N):

(M) 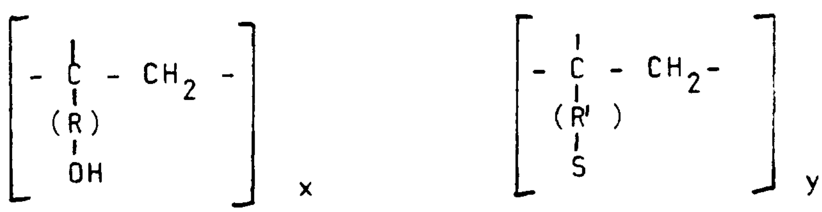

(N) 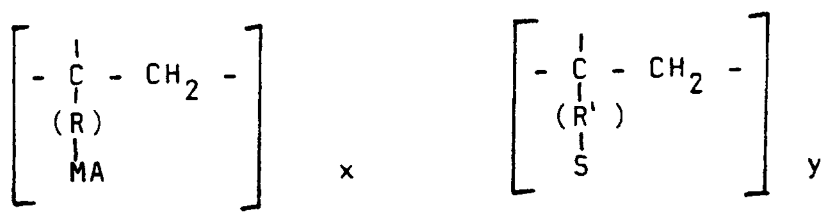

Beim Aufbau der Grundgerüste können auch Einheiten mit einpolymerisiert werden, die weder Säuregruppen tragen noch mit einer polymerisierbaren Gruppe versehen werden. Zum einen kann es nützlich sein, auf diese Weise die Löslichkeit zu verändern, wie z.B. beim Einbau von inerten Methylmethacrylat-Einheiten (O):

(O) 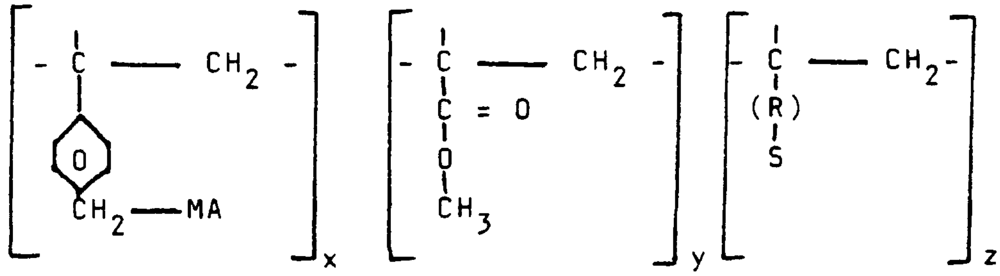

Zum anderen kann der Einbau von zusätzlichen Einheiten mit z.B. Halotriazin-, Epoxid-, Isocyanat- oder Aldehydgruppen (P) zum Zwecke einer zusätzlichen Reaktion mit dem Collagenanteil von Zahn- oder Knochensubstrat von Nutzen sein.

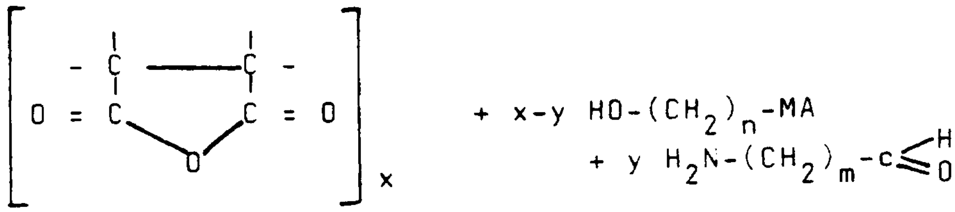

(P) 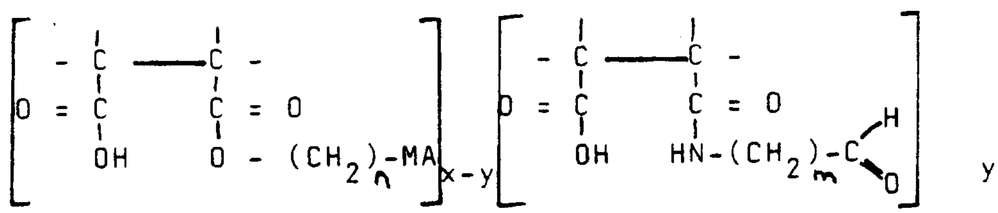

Auch der Einbau von Einheiten mit Gruppen, die Teile eines Polymerisations-Katalysatorsystems sein können, kann von Vorteil sein, wobei diese Gruppen nicht unbedingt bei der Homo- oder Co-Polymerisation des Grundgerüstes als solche Gruppen vorhanden sein müssen, sondern auch nachträglich z.B. über eine Halogen-, Alkohol-, Anhydrid- oder Aminfunktionelle Gruppe angeknüpft werden können.

Die Art der Reaktionsführungen, die zu den oligomeren oder prepolymeren Grundverbindungen führen, kann durch die Wahl des Lösungsmittels, der Löslichkeiten, der Konzentration, der Temperaturen und die Wahl der Polymerisationskatalysatoren bestimmt werden und sind dem Fachmann bekannt.

Wichtig kann sein, daß die benutzten Polymerisationskatalysatoren durch die Reaktion selbst zerstört werden oder eventuelle Reste von diesen vor dem Anknüpfen der polymerisierbaren Gruppen an die Grundgerüste entfernt werden.

Die oligomeren Verbindungen haben vorteilhafterweise ein Molekulargewicht von gößer als 500, die prepolymeren Verbindungen haben vorteilhafterweise ein Molekulargewicht von gößer als 1.500, bevorzugt nicht größer als 100.000, besonders bevorzugt nicht größer als 20.000.

Die erfindungsgemäßen Mischungen können auch noch andere polymerisierbare ungesättigte Monomere und / oder Oligomere und / oder Prepolymere enthalten, die keine Säuregruppen und / oder deren Salze und / oder deren reaktive, leicht hydrolysierbare Säurederivatgruppen aufweisen. Besonders eignen sich jene Monomere, die Bestandteile üblicher Composites sind, wie z.B. Bis-Gma oder Triethylenglykoldimethacrylat. Ebenso können die Mischungen gegebenenfalls noch andere Verbindungen enthalten, die zwar Säuregruppen und / oder deren Salze und / oder deren reaktive leicht hydrolysierbare Säurederivatgruppen aufweisen, aber keine Gruppen enthalten, die ungesättigt und polymerisierbar sind. Dabei werden mehrbasische Säuren bzw. deren reaktive, leicht hydrolysierbare Derivate bevorzugt. Besonders bevorzugte mehrbasische Säuren sind Hydroxysäuren wie Weinsäure oder Citronensäure, aber auch Polysäuren wie Polycarbonsäuren, Polyphosphorsäuren oder Polyphosphonsäuren oder auch Polysulfonsäuren.

Auch Verbindungen mit chelatbildenden Gruppen, die nicht Säure- oder leicht hydrolysierende Säurederivatgruppen enthaltende Verbindungen sind, können zugesetzt sein. Beispiele solcher Art sind Vanillate, Syringate und Salicylate.

Der Anteil von polymerisierbaren, säuregruppen- oder reaktiven, säurederivatgruppenhaltigen Verbindungen an der Gesamtmenge von polymerisierbaren Verbindungen liegt in besonders bevorzugten Mischungen zwischen 20% und 60%, in bevorzugten Mischungen zwischen 5% und 100%, jedoch zeigen auch Mischungen unter 5% noch deutliche Effekte, die den erfindungsgemäßen Mischungen zu eigen sind. Dabei kann z.B. vorteilhaft sein, die Menge oder einen Teil der Menge der polymerisierbaren Verbindungen, die die reaktiven Säure- oder Säurederivatrestegruppen enthalten, vor der Einarbeitung der reaktiven Füllstoffe in die Mischung auf diese aufzuziehen und eventuell durch Spuren von Wasser schon mit der Oberfläche (ionisch) reagieren lassen, ohne daß jedoch die polymerisierbaren Doppelbindungen schon reagieren. Dieses soll dann erst nach Einmischen dieser vorbehandelten Füllstoffe in der Gesamtmischung und beim Polymerisieren derselben geschehen.
Als reaktive Füllstoffe der erfindungsgemäßen Compositezementmischungen, die mit den in den polymerisierbaren Verbindungen enthaltenden Säuregruppen oder Säurederivatgruppen reagieren können, eignen sich die reaktiven Füllstoffe gemäß Anspruch 1.

Die reaktiven Füllstoffe müssen nicht unbedingt als alleinige Füllstoffe in den erfindungsgemäßen Mischungen enthalten sein. Es können auch andere, für Composites übliche, im Sinne von Ionenreaktionen bzw. Zementabbindereaktionen nicht reaktive organische oder anorganische, besonders silanisierte Füllstoffe zugemischt sein. Dieses kann z.B. von Vorteil sein, wenn die gehärteten Produkte höhere mechanische oder chemische Widerstandsfähigkeit aufweisen sollen. Weiterhin kann es erforderlich sein, die erfindungsgemäßen Mischungen zum Zwecke der Lagerung in getrennte Komponenten aufzuteilen, wobei z.B. eine Komponente die polymerisierbaren Monomeren mit Säuregruppen und nicht reaktivem Füllstoff, die andere Komponente polymerisierbare Monomere ohne Säuregruppen und reaktiven Füllstoff enthält.
Der Anteil der reaktiven Füllstoffe am Gesamtfüllstoff beträgt in bevorzugten Mischungen mehr als 5 Gew.-%, in besonders bevorzugten Mischungen mehr als 30 Gew.-%. In einigen Fällen können jedoch auch geringe Anteile einen deutlichen Effekt, wie z.B. Alkalität, zeigen, was bei Füllungsmaterialien im Dentinbereich wichtig sein kann. Der Gesamtfüllstoffgehalt der erfindungsgemäßen Mischungen liegt besonders zwischen 10% und 95%, bevorzugt zwischen 30% und 85% (Gew.-%) der Gesamtmischung.
Als Härtungsmittel eignen sich prinzipiell alle diejenigen Systeme, die eine radikalische Polymerisation von olefinischen Verbindungen auslösen können. Dabei ist es nicht wesentlich, ob die Katalysatorreaktion durch Erhitzen, durch Hinzufügen eines den Katalysator zur Reaktion bringenden Aktivators oder durch Bestrahlen mit Licht initiiert wird. Vielmehr ist wichtig, daß das Katalysatorsystem ausreichend löslich in der Mischung ist und nicht wesentlich durch die Säure- oder Säurederivatgruppen enthaltenden polymerisierbaren Verbindungen blockiert oder zum Zerfall gebracht wird.

Bevorzugt werden für lichthärtbare Mischungen Härtungssysteme aus ∝-Diketonen und tertiären Aminen, wie sie z.B. in der FR-PS 2 156 760 beschrieben sind, oder Kombinationen von Sulfinsäuresalzen und Xanthonen oder Thioxanthonen, wie sie z.B. in der EP 0 132 318 beschrieben sind.
Im Falle von 2-Komponenten-Mischungen eignen sich besonders Kombinationen von einer Komponente, die organische Peroxide enthält und einer anderen Komponente, die ein tertiäres Amin und / oder eine Verbindung, die Schwefel in der Oxidationsstufe +2 oder +4 aufzeigt, enthält. Die Komponente, die nicht Peroxid enthält, kann auch bevorzugt chelatbildende 2-wertige Metallionen enthalten, insbesondere Calciumionen.
Besonders bevorzugt sind Benzoylperoxid als organisches Peroxid und Natriumbenzolsulfinat oder Natriumparatoluolsulfinat als Schwefelverbindung.

Eine besonders vorteilhafte Mischung liegt vor, wenn der Komponente, die das Natriumsulfinat enthält, zum ausreichenden Lösen dieses Salzes Alkohole zugegeben sind, die eine oder mehrere polymerisierbare olefinisch ungesättigten Gruppen enthalten.
Dabei eignen sich z.B. Hydroxyalkylmethacrylate wie Hydroxyethylmethacrylat oder Hydroxygruppen tragende Vinylverbindungen wie Allylalkohol, und besonders hydroxylgruppenhaltige Dimethacrylatverbindungen wie Bisphenol-A-glycidylmethacrylat oder Glycerindimethacrylat oder hydroxylgruppenhaltige Divinylverbindungen wie Glycerindiallylether. In der Regel ist eine Zugabe von 10 bis 20 Gew.-% dieser hydroxylgruppenhaltigen polymerisierbaren Monomeren notwendig.

Weiterhin können Härtungsmittel zugesetzt werden, die für Zemente typisch sind und die Ionenreaktionen beschleunigen können, wie z.B. Wasser oder auch Weinsäure oder Mellithsäure.

Die erfindungsgemäßen Compositezemente können natürlich noch übliche Kunststoffzusätze enthalten, die das Aussehen und die Stabilität der noch nicht gehärteten Pasten oder der ausgehärteten Produkte in bekannter Weise günstig beeinflussen wie Pigmente` UV-Stabilisatoren, Antioxidantien und ähnliches.
Ebenso können geringe Mengen Schwermetallsalze wie z.B. von Fe, Cu, Mn, Co, Sn, Cr., Ni und Zn zugegeben sein, die sich günstig auf das Haftverhalten z.B. an Zahnsubstanzen auswirken.
Zusätze von heilenden Komponenten wie z.B. Cortison oder Cortikoide oder Klauenöl können zugegeben

werden, sind dann aber nicht aus chemisch-physikalischen, sondern aus rein medizinischen Gründen indiziert. Der Compositezement dient dann lediglich außer seiner eigentlichen Funktion als Zement und gegebenenfalls als Calciumionen- oder ph-Milieuspender auch als pharmazeutisches Depot. Aus ähnlichen Gründen können auch fluoridspendende Verbindungen, wie z.B. Na-Fluorphosphat oder Aminfluoride zugesetzt sein.

Die erfindungsgemäßen Compositezemente zeigen zum Teil Eigenschaften, die in ihrer Ausgeprägtheit bisher einerseits nur Composites oder andererseits nur Zementen zu eigen waren.

So zeigen sie in den meisten Fällen bei guter Bruch- und Kantenfestigkeit, guter Härte und wenig Sprödigkeit eine gute Gewebeverträglichkeit und eröffnen sogar Möglichkeiten stark alkalisch reagierender Kunststoffüllungen. Außerdem sind sie bedeutend weniger löslich als Zemente, die einen vergleichsweisen Füllstoff enthalten. Mit Kunststoffüllungsmaterialien auf Basis polymerisierbarer ungesättigter Monomerer sind sie copolymerisierbar und geben einen festen chemischen Verbund.

Andererseits haften sie über Ionenreaktionen auch chemisch fest auf Zementen und an der Zahnsubstanz.

Auch ist es möglich, lichthärtende Compositezemente zu erhalten

Nicht zuletzt können auch Zusätze von Paraffinöl, Polymeren, Leinöl, oberflächenaktiven Substanzen usw., wie sie bei einigen Zementtypen aus Gründen der Konsistenz oder Löslichkeit notwendig sind, praktisch entfallen.

Die Säuregruppen oder Säurederivatgruppen enthaltenden polymerisierbaren Verbindungen wirken meist selbst als oberflächenaktive Substanzen und benetzen die anorganischen Füllstoffe ausgezeichnet.

Eine interessante Eigenschaft der neuen Composite-Zemente ist, daß beim Abbindevorgang einerseits ein Polymerisationsschrumpfungsprozeß stattfindet, dieser Prozeß aber andererseits durch eine gleichzeitige oder nachträgliche Wasseraufnahme (z.B. Hydratationsvorgänge) wieder rückgängig gemacht oder sogar überkompensiert werden kann. Dieser Prozeß ist über die in der unpolymerisierten Mischung vorhandenen Wassermenge steuerbar und auch von der Feuchte des umgebenden Mediums nach der Polymerisation abhängig.

Die erfindungsgemäßen Compositezemente eignen sich je nach Art der Composition für Wurzelkanalfüllungen, Pulpenüberkappungen, Unterfüllungen, als gefüllter Kavitätenliner und als Füllungsmaterial, weiterhin auch als Zemente für Kronen und Brücken, für orthodontische Klebungen, als Schutzfilm für angeätzten Schmelz, als Haftopaker, als härtbare Mischungen zum Ausfüllen, Versiegeln und Kleben von oxidischen, mineralischen, glasartigen, keramischen, metallischen und biologischen Substraten, als haftvermittelnde Schicht zwischen oxidischem, mineralischem, glasartigem, keramischem, metallischem oder biologischem Substrat und radikalisch polymerisierbaren Kunststoffmaterialien und als haftvermittelnde Zwischenschicht von Zementen zu Composites. Nicht zuletzt können auch polymerisierbare, harte und sehr bruchfeste Dentalgipse bereitet werden. Des weiteren ergeben sich Anwendungsmöglichkeiten als Knochenzemente, aber auch als Zement für allgemeine Zwekke.

Die erfindungsgemäßen Compositezemente eignen sich auch zur Herstellung von Formkörpern, wobei diese direkt oder auch in Verbindung mit anderen Werkstoffen, wie z.B. im Verbund mit Metallgerüsten, verwendet werden können. Besonders, wenn die Formkörper Ca-Verbindungen enthalten, können sie als Implantate sehr geeignet sein.

Nachstehende Präparate und Beispiele dienen der weiteren Erläuterung der Erfindung. Wenn nichts anderes angegeben ist, beziehen sich Teile und %-Angaben auf das Gewicht.

Präparat 1

Herstellung einer polymethacrylierten Oligomaleinsäure:

260 g Maleinsäureanhydrid wurden mit 2.000 ml Toluol und 40 g Benzoylperoxid unter Rückfluß 6 Tage erhitzt. Es bildete sich ein bräunlich oranger Niederschlag. Nach Beendigung der Reaktion wurde die überstehende Toluol-Lösung dekantiert, der Rückstand mit Hexan gewaschen. Ausbeute: 200 g. Diese 200 g wurden mit der gleichen Menge Tetrahydrofuran versetzt.

Eine Molekulargewichtsbestimmung ergab ein mittleres Molekulargewicht von 439, was in etwa einem Oligomerisationsgrad von 4 Maleinsäureanhydrid-Einheiten entspricht. Das IR-Spektrum zeigte die C = O-Bande von Anhydrid-Gruppen (1.790 $cm^{-1}$), jedoch keine Säure-OH und keine Doppelbindungen. 100 g der Tetrahydrofuranlösung des Oligomaleinsäureanhydrids wurden mit 10 g Zinkstaub versetzt, gerührt und wieder abfiltriert, wodurch die Farbe der Lösung deutlich hell wurde.

Nach Zugabe von 60 g Hydroxyethylmethacrylat und katalytischen Mengen Orthophosphorsäure wurde der Ansatz für 2 Wochen stehengelassen. Die Mischung wurde deutlich viskoser. Nach Abziehen der flüchtigen Komponenten im Vakuum und Waschen in Hexan erhielt man ein viskoses Öl, das sehr gut in

Aceton, aber auch in TEDMA und Bis-GMA löslich war.

Das IR-Spektrum zeigte, daß die Anhydrid-C = O-Bande fast verschwunden war, eine Säure-OH-Bande und auch eine Doppelbindungsbande deutlich erschienen war.

Präparat 2

Herstellung einer polymethacrylierten Polycarbonpolyphosphonsäure:

100 g der Tetrahydrofuranlösung des Oligomaleinsäureanhydrid von Präparat 1 wurden mit Zinkstaub gerührt und mit 30 g Hydroxyethylmethacrylat versetzt.

Nachdem die Mischung zwei Wochen bei Raumtemperatur reagiert hatte, wurden 40 g Hydroxy-ethan-1,1-diphosphonsäure hinzugelöst und weitere zwei Wochen stehengelassen. Nach Abziehen des Tetrahydrofurans erhielt man eine recht viskose Flüssigkeit, die mit Hexan gewaschen wurde. Das IR-Spektrum zeigte C = C-Banden bei 1.640 $cm^{-1}$, P(O)OH-Banden bei 1.200 $cm^{-1}$. Die Substanz reagiert sauer und vergelt bei Zugabe von Activator/Peroxid.

Präparat 3

Herstellung einer prepolymeren polymethacrylierten Polymaleinsäure:

60 g Maleinsäureanhydrid und 9 g Lauroylperoxid wurden in 150 ml Tetrahydrofuran vier Tage unter Rückfluß gekocht, das Tetrahydrofuran wieder abgezogen und das viskose Öl mit Hexan gewaschen (Polymaleinsäureanhydrid, Molekulargewicht 1.850, entsprechend ca. 17 Einheiten, IR-Spektrum identisch mit dem des Oligomaleinsäureanhydrid als Vorstufe für Präparat 1.

9,8 g des Öls wurden in 30 ml THF gelöst und mit 12 g Hydroxymethacrylat zwei Wochen gerührt. Nach Abziehen des THF erhielt man ein viskoses Öl von polymethacrylierter Polymaleinsäure, dessen IR-Spektrum identisch mit dem der polymethacrylierten Oligomaleinsäure des Präparats 1 war.

Präparat 4

Herstellung eines prepolymeren polymethacrylierten Polychlorophosphates:

42 g Hydroxyethylmethacrylat und 8 g Lauroylperoxid wurden in 400 ml Toluol gelöst, 1 h bei 65° C gehalten. Das entstandene Pulver wurde abfiltriert, mit Hexan gewaschen und getrocknet.

Ausbeute: 40 g Polyhydroxyethylmethacrylat (Poly-HEMA) Molgewichtsbestimmung: 5.700 ≙ ca. 44 Monomereinheiten IR-spektroskopisch identisch mit höhermolekularem Poly-HEMA-Produkt der Firma Aldrich

13 g des selbst hergestellten Poly-HEMA wurden mit 8 g Methacrylsäurechlorid und 8 g Triethylamin 3 Tage gerührt, der Niederschlag mit Wasser gewaschen und getrocknet.

Ausbeute: 15 g teilmethacryliertes Poly-HEMA

3,3 g dieses Pulvers werden in 50 ml Tetrahydrofuran mit 1,5 g Phosphorylchlorid versetzt und 4 Tage bei Raumtemperatur gerührt. Nach Filtration des Niederschlages und Waschen mit Hexan werden 3,8 g eines weißen Pulvers erhalten, welches IR-spektroskopisch gut mit einem polymethacrylierten Produkt mit $-O-P(O)Cl_2$-Gruppen in Einklang gebracht werden kann. Das Produkt zeigt kaum noch C-OH-Banden, jedoch weiterhin die C = O (1.730 $cm^{-1}$) und C = C-Banden (1.640 $cm^{-1}$) der Methacrylgruppe sowie neu aufgetretene Banden im Bereich P-O-Alkyl (1.030 $cm^{-1}$).

Präparat 5

Herstellung eines polymethacrylierten Polysulfonats:

5,4 g Hydroxyethylmethacrylat, 10,1 g Kaliummethacryloylpropylsulfonat und 1,6 g Lauroylperoxid wurden in 80 ml Methanol und 20 ml Toluol bei 65° C erhitzt, bis die Niederschlagsbildung beendet war. Es wurde filtriert, mit Hexan gewaschen und getrocknet.

Ausbeute: 6,4 g wasserlösliches Copolymerisat, Molgewicht 7.490, entsprechend ca. 20 Einheiten von jedem der beiden eingesetzten Monomere.

1,88 g des Copolymerisates wurden mit 0,54 g Methacrylsäurechlorid und 0,50 g Triethylamin in 50 ml Tetrahydrofuran vier Tage bei Raumtemperatur gerührt. Der entstandene Niederschlag wurde mit Hexan

gewaschen und getrocknet.

Ausbeute: 1,92 g weißes, wasserlösliches, pulveriges Polymethacrylpolykaliumsulfonat, welches im IR deutlich wieder C=C bei 1.640 $cm^{-1}$ zeigt. Gehalt an Kalium: 7,9 %, Gehalt an Schwefel: 6,2 %.

Präparat 6

Herstellung von polymethacrylierter Polyborsäure:

3,3 g teilmethacryliertes Poly-HEMA aus Präparat 4 wurden mit 3,1 g Borsäure und 4,1 g Phosphorsäure so lange auf 80° C in Dioxan erhitzt, bis die Niederschlagsbildung abgeschlossen war.
Nach Filtrieren wurde mit Wasser phosphat- und borsäurefrei gewaschen, getrocknet und 3,45 g einer leicht bräunlichen polymethacrylierten Polyborsäure erhalten.

IR-spektroskopisch sind die C = C-Banden und C = O-Banden unverändert, es treten neue Banden (B-OH) bei 3.220 $cm^{-1}$ im Spektrum auf. Der Borgehalt ergibt sich zu 2,4 Gew.-%.

Beispiel 1

Herstellung eines 2-Komponenten-Compositezements auf der Basis von halophosphoryliertem Bis-GMA und Phosphatzement pulver (ZnO/MgO):

Es wurden verschiedene 2-Komponenten-Mischungen wie folgt hergestellt und zur Reaktion gebracht.
I. Für die erste Komponente wurde eine Harzmischung aus 10 Teilen Bisphenol-A-glycidylmethacrylat (Bis-GMA) 10 Teilen Triethylenglycoldimethacrylat (TEDMA) und 1 Teil Phosphorylchlorid hergestellt und fünf Tage bei Raumtemperatur stehengelassen (Harz 1, polymerisierbare Halophosphorsäureverbindung, entsprechend EP 0058 483).
Ein Teil von Harz 1 wurde mit 1% Benzoylperoxid als Katalysator versetzt (Harz 2).
Eine Katalysatorpaste wurde hergestellt durch inniges Vermischen von 22 Teilen Harz 2 und 78 Teilen feinstgemahlenem silanisierten Bariumglas (nicht reaktiver Füllstoff).
Diese Mischung hatte eine dick-pastöse Konsistenz.
II. Für die zweite Komponente wurde eine Harzmischung (Harz 3) hergestellt aus

50 Teilen Bis-GMA } (polymerisierbare nicht säuregruppenhaltige Verbindungen)
50 Teilen Glycerindimethacrylat }
1 Teil N,N-Bishydroxyethyl-para-toluidin
3 Teilen Natriumbenzolsulfinat
1 Teil Wasser und
0,002 Teilen Eisenoxalat.

Eine Aktivatorpaste wurde hergestellt durch Vermischen von 24 Teilen Harz 3 und 76 Teilen der Pulverkomponente vom "Harvard"-Phosphatzement *der Fa. Hoffmann und Richter, Berlin (reaktiver Füllstoff).
Nach Vermischen der Katalysatorpaste mit der Aktivatorpaste härtete das Material innerhalb von 1 bis 2 Minuten aus.
Nach ca. 30 Minuten wurde eine Barcolhärte von 54 gemessen. Die Druckfestigkeit betrug nach 24 Std. Naßlagerung bei 37° C 1.800 $kg/cm^2$.
Der erfindungsgemäße Zement erwies sich als sehr gewebeverträglich und ließ sich gut als Knochenzement verwenden.

* Phosphatzementproduckt auf der Basis von Zinkoxid und Magnesiumoxid

Beispiel 2

Als Vergleich zum Beispiel 1 wurde die Pulverkomponente von "Harvard"-Phosphatzement * mit der Carboxylatflüssigkeit des Produktes "Carboxylat-Zement" (Voco-Chemie, Cuxhaven) zum Erhärten gebracht. Die Erhärtungszeit lag in dem für Carboxylatzemente üblichen Zeitraum von 5 bis 8 Minuten.
Nach ca. 30 Minuten war ein Barcolhärtewert kaum zu erhalten. Der Meßstift drang in den Formkörper ohne großen Widerstand ein und zerbrach ganz leicht. Nach 3 Stunden wurde ein geringer Wert angezeigt, der unter 5 lag. Das Material war weiterhin sehr zerbrechlich. Die Druckfestigkeit nach 24 Stunden Naßlagerung bei 37° C betrug 550 kg/cm$^2$. Diese Größenordnung fand man auch bei anderen klassischen Carboxylatzementen. Auch verbesserte Produkte zeigten bei Zusätze inerter Füllstoffe wie Quarzpulver oder von Polymeren oder von Chelatbildnern zum Zement nur Druckfestigkeiten von 800-900 kg/cm$^2$.

Beispiel 3

Härtender 2-Komponenten-Kompositzement auf Basis von halophosphoryliertem Bis-GMA und Ionomerzementpulver:

Eine Aktivatorpaste wurde durch Zugabe von 7 Teilen Ionomerzementpulver "Fuji"* (G-C Dental Corp., Japan) zu
3 Teilen Harz 3 aus Beispiel 1 bereitet. Nach Vermischen dieser Paste mit gleichen Teilen einer Katalysatorpaste aus Beispiel 1 härtete das Material schnell aus. Nach 30 Minuten wurden eine Barcolhärte von 57 gemessen. Die Druckfestigkeit nach 24 h/37° C betrug 2.100 kg/cm$^2$. Eine Löslichkeit konnte nach 24stündiger Lagerung im Wasser von 37° C nicht festgestellt werden.

Beispiel 4

Zwei durch Mischen härtbare Pasten auf Basis von Polymethacrylpolycarbonsäure und Phosphatzementpulver:

Es wurden zwei durch Mischen härtbare Pasten hergestellt. Für die erste Paste wurde ein Gemisch aus
90 Teilen Triethylenglykoldimethacrylat
7 Teilen Polymethacrylpolycarbonsäure gemäß Präparat 1 und 2 Teilen Benzoylperoxid hergestellt.

Dieses Harz 4 wurde mit silanisiertem amorphen gesinterten Siliciumdioxid zu 68% gefüllt und ergab die Katalysatorpaste.

Für die zweite Paste wurde ein Gemisch aus
50 Teilen Bis-GMA
50 Teilen Hydroxyethylmethacylat
1 Teil N,N-Bishydroxyethylparatoluidin sowie
3 Teilen Natriumbenzolsulfinat
mit Phosphatzement angeteigt.
Der Füllstoff dieser Aktivatorpaste betrug 75%. Zum Aushärten wurden Aktivator- und Katalysatorpaste vermischt.

Der gehärtete Compositezement hatte eine Druckfestigkeit von 1.500 kg/cm$^2$.
Die Barcolhärte betrug 5l. Das Material zeigte im Streßtest (Wassertauchbäder im Wechsel von 0° C und 60° C) nach 4.000 Zyklen keine Ermüdungserscheinungen. Im Gegenteil, die Barcolhärte stieg auf 59 und das Material war äußerst kantenfest. Es haftete sehr gut an Dentin und Schmelz von Rinderzähnen.

Beispiel 5

Bereitung eines lichthärtenden Composite-Ionomerzementes:

Auf 30 g der Pulverkomponente des Ionomerzements "Ceramfil Alpha" (Fa. PSP Dental, Belvedere, Kent, U.K.) werden mit Hilfe einer ethanolischen Lösung 5 g einer Mischung von
50 Teilen Bis-GMA
50 Teilen Triethylenglycoldimenthacrylat
20 Teilen polymethacrylierte Polymaleinsäure (Präparat 3)
aufgezogen und getrocknet.
5 g dieses Pulvers werden mit 5 g eines mit Polyacrylsäure belegten Ionomerpulvers des Produktes

"Ceramfil Beta, Aqua Set" (Fa. PSP Dental) innig vermischt und bilden das neue Ionomerzementpulver (I) für den lichthärtenden Ionomerzement.

Eine Flüssigkeit (II) wird bereitet aus:

15 g $H_2O$
10 g Hydroxyethylmethacrylat
0,15 g N,N-Bishydroxyethyl-p-toluidin
0,45 g Natriumbenzolsulfinat.
0,08 g Campherchinon

Das Ionomerzementpulver (I) wird mit der Flüssigkeit (II) bis zu einer pastösen Konsistenz (ca. 4 Teile Pulver, 1 Teil Flüssigkeit) gemischt und ein Teil der Mischung im Dunklen stehengelassen (A), ein anderer Teil mit Halogenlicht einer Lampe der Fa. Litema, Deutschland ("HL 150") ausgehärtet (B).

Die Aushärtung im Fall (A) beginnt nach 10 min und ist nach 20 min beendet. Im Falle (B) ist die Mischung in einer Schichtdicke von 2 mm nach 40 sec gut ausgehärtet.

Nach der Aushärtungsmethode (A) und (B) werden jeweils Probekörper entsprechend DIN-Norm 13922 hergestellt und nach 3 h die Biegefestigkeit gemessen. Ebenso werden vom Glas-Ionomerzement "Ceramfil Beta, Aqua Set" nach Anmischen (Pulver, $H_2O$) Probekörper hergestellt (Erhärtungsbeginn 5 min, gut durchgehärtet nach 10 min) und nach 3 h und 24 h die Biegefestigkeit gemessen.

| Biegebruchfestigkeiten (N/mm$^2$): | | |
|---|---|---|
| Lichthärtender Ionomerzement (Lichthärtung) | Lichthärtender Ionomerzement (Dunkelhärtung) | Ionomercement Ceramfil Beta Aqua Set |
| 11,6 ( 3h)<br>43,0 (20h) | 6,3 ( 3h)<br>8,8 (20h) | 12,1 ( 3h)<br>13,2 (20h) |

<u>Beispiel 6</u>

Lichthärtender Ionomer-Zement:

Auf 1,75g Ionomerzementpulver des Produktes Ceramfil Beta, Aqua Set, wird eine Mischung von 1,50g polymethacryliertem Polychlorophosphat (Präparates 4) und 0,25g Triethylenglycol als ethanolische Lösung aufgezogen und getrocknet. (Pulverkomponente) Als Reaktionspartner wird eine Mischung von

150 Teilen Hydroxyethylmethacrylat
50 Teilen Wasser
1 Teil Campherchinon
2 Teilen N, N-Bishydroxyethyl-p-toluidin
5 Teilen Natriumbenzolsulfinat

bereitet (Flüssigkeitskomponente).

4 Teile Pulverkomponente und 1 Teil Flüssigkeitskomponente werden zu einer pastösen Konsistenz vermischt und ein Teil mit Halogenlicht gehärtet (20sec), der andere Teil im Dunklen stehen gelassen (ca. 50 min bis zur Erhärtung).

Die halogenlicht gehärtete Probe zeigte nach 3 h eine Biegebruchfestigkeit von 18,4 N/mm$^2$, nach 20 h 27,2 N/mm$^2$.

<u>Beispiel 7</u>

Zahnwurzelkanalfüllungsmaterial

Eine Paste, bestehend aus

12 Teilen Hydroxyethylmethacrylat
11 Teilen Glycerindimethacrylat
1 Teil N, N-Bishydroxyethyl-p-toluidin
und 76 Teile Ionomer-Pulver des Produktes Ceramfil Alpha
wurden mit einem Harz aus
5 Teilen polymethacrylierter Polyborsäure (Präparat 6 )
50 Teilen BIS-GMA

45 Teilen Triethylenglycoldimethacrylat
und 3 Teilen Benzoylperoxid
zu gleichen Teilen gemischt und in einen geleerten Zahnwurzelkanal gefüllt. Nach 2 min ist die Mischung fest. Der Zahn wird 14 Tage in ein Methylen blau -Bad gelegt, längs aufgeschnitten und beschliffen. Es konnte kein Eindringen von Farbstoffs zwischen Zahnsubstanz und Füllungsmaterial festgestellt werden.

Beispiel 8

Klebezement

Vom Silikatzementpulver des Produktes "Omnifil" (Fa. Jota, Deutschland) wurde eine Korngrößenfraktion von kleiner als 20μ gesiebt und 100 Teile davon mit 0,2 Teilen Natriumbenzol-Sulfinat und 0,4 Teilen N,N-Bis-hydroxyethyl-p-toluidin, 7 Teilen BIS-GMA und 8 Teilen Triethylenglycoldimethacrylat zu einer Paste (I) gemischt.
Auf weitere 100 Teile vom gesiebten Produkt werden
5 Teile Benzoylperoxid
aufgezogen und diese ebenfalls mit
7 Teilen BIS-GMA und
8 Teilen Triethylenglycoldimethacrylat
zu einer Paste (II) gemischt.

Eine Flüssigkeit (III) wurde hergestellt aus
13 Teilen Phosphorsäure
15 Teilen Hydroxyethylmethacrylat
60 Teilen Wasser
12 Teile polymethacryliertes Polysulfonat (Präparat 5)

Von den Pasten I und II sowie von der Flüssigkeit III wurden jeweils 1 Teil genommen, zusammen gemischt und zwischen angeätztem Zahnschmelz und einem Metallplättel aus Resilloy (Fa. Renfert. Deutschland) gegeben. Auf das Metallplättchen wurde leichter Druck ausgeübt. Nach 2 min war die Klebemischung fest.

Nach 3 Std. wurde mit Hilfe einer Zugkraft-Testmaschine die geklebte Probe bei einer Belastung von 5,6 $N/mm^2$ wieder auseinandergerissen.

Beispiel 9

Expandierender lichthärtender Kavitätenliner

Ein lichthärtender Kompositzement wird hergestellt aus
50 Teilen BIS-GMA
50 Teilen Triethylenglycoldimethacrylat
1 Teil Campherchinon
1 Teil Butyldimethylanilin
10 Teilen polymethacrylierter Polymaleinsäure (Präparat 3)
40 Teile mikrofeiner, silanisierte Kieselsäure
240 Teilen Ionomerzement-Pulver des Produktes Ceramfil Beta, Aqua Set

Der Kompositzement ist nach 20 sec Halogenlichtbestrahlung bis zu einer Schichtdicke von 3mm voll ausgehärtet. Die Polymerisationsschrumpfung in Wasser mißt sich zu 0,0% nach 10 min,nach 30 min zeigt der Probekörper eine Expansion von 0,24% und 0,80% nach 16 h. Dieser Wert verändert sich dann kaum noch.
Mit diesen Eigenschaften eignet sich dieser Kompositzement vorzüglich als Kavitätenliner unter einem Zahnfüllungsmaterial.
Er kann einen großen Teil der Polymerisationsschrumpfung des Composite-Füllungsmaterials durch seine Expansionseigenschaften auffangen.

Beispiel 10

Ein polymerisierbarer Gips wurde dadurch hergestellt, daß man 90 Teile Moldano-Gips (Bayer), mit Hilfe einer alkoholischen Lösung mit 10 Teilen von Harz 1 des Beispiels 1 belegte und das Lösungsmittel abzog.

Das so erhaltene Gipspulver wurde mit einer Mischung aus 25 Teilen Wasser,das mit 1% N,N-Bishydroxyethylparatoluidin und 3% Natriumbenzolsulfinat versetzt war, und 75 Teilen Hydroxyethylmethacrylat bis zu einem fließenden Brei versetzt.
Dieser wurde in eine Form gegossen und war nach ca. 2 Min. ausgehärtet.
Nach Entfernen der Form erhielt man einen ausgezeichneten Modellformkörper, der kaum zerbrechlich war und eine ausgezeichnete Kratzfestigkeit aufwies.

Beispiel 11

Man verfuhr nach Beispiel 10 vermischte jedoch den unbehandelten Gips mit Wasser zu einem fließenden Brei. Dieser Brei benötigte 30 Minuten zum Abbinden und der dabei entstehende Formkörper war sehr zerbrechlich und wenig kratzfest.

**Patentansprüche**

**1.** Polymerisierbare Zementmischungen, enthaltend
  a) polymerisierbare, ungesättigte Monomere und/oder Oligomere und/oder Prepolymere, die Säuregruppen und/oder deren reaktive Säurederivatgruppen enthalten,
  b) feinteilige, reaktive Füllstoffe, die mit diesen Säuren oder Säurederivaten reagieren können nämlich Pulver von Phosphatzement (ZnO/MgO), Silikatzementen, Ionomerzementen oder von ionen austauschenden Zeolithen sowie
  c) Härtungsmittel,
dadurch gekennzeichnet, daß die Komponenten a) und b) derart ausgewählt sind, daß die Säuregruppen oder Säurederivatgruppen gemäß a) mit den feinteiligen, reaktiven Füllstoffen gemäß b) ionisch zu einer Zementreaktion zu führen vermögen.

**2.** Mischungen nach Anspruch 1, dadurch gekennzeichnet, daß die polymerisierbaren Verbindungen mindestens zwei polymerisierbare Gruppen und mindestens zwei Säuregruppen bzw. deren reaktive Derivatgruppen enthalten.

**3.** Mischungen nach Anspruch 1, dadurch gekennzeichnet, daß die polymerisierbaren Verbindungen drei oder mehr polymerisierbare Gruppen und drei oder mehr Säuregruppen bzw. deren reaktive Derivatgruppen enthalten.

**4.** Mischungen nach einem der Ansprüche 1 bis 3, dadurch gekennzeichnet, daß die polymerisierbaren ungesättigten Verbindungen Acryl-, Methacryl-, Vinyl-, Styryl- oder eine Mischung dieser Gruppen enthalten.

**5.** Mischung nach Anspruch 1 bis 3, dadurch gekennzeichnet, daß die polymerisierbaren ungesättigten Verbindungen Acryl- oder Methacrylgruppen enthalten.

**6.** Mischungen nach einem der Ansprüche 1 bis 5, dadurch gekennzeichnet, daß die Säuregruppen Carbonsäurereste oder deren Salze, Phosphorsäurereste der Formeln

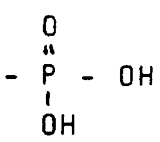 , 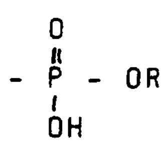 , 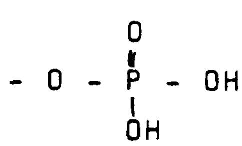 , 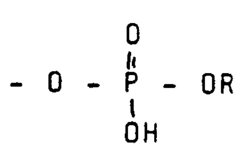

oder deren Salze, wobei R Alkyl, Aryl oder Vinyl bedeutet,
Schwefelsäurereste der Formeln $-SO_2H$, $SO_3H$, $-O-SO_3H$ oder deren Salze oder
Borsäurereste der Formeln

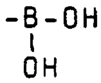 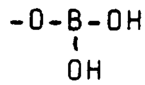 bzw. 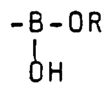 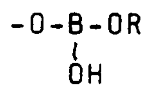

oder deren Salze, wobei R Alkyl, Aryl, Vinyl bedeutet, sind.

**7.** Mischungen nach einem der Ansprüche 1 bis 6, dadurch gekennzeichnet, daß die reaktiven Säurederivatgruppen in Form von Säurehalogeniden oder, - anhydriden.

**8.** Mischungen nach Anspruch 1 bis 7, dadurch gekennzeichnet, daß das ungesättigte Monomer ein Halophosphorsäureester des Bis-GMA ist.

**9.** Mischungen nach Anspruch 1 bis 7, dadurch gekennzeichnet, daß die Oligomeren oder Prepolymeren solche Verbindungen sind, die die polymerisierbaren ungesättigten Gruppen und die Säurereste, deren Salze oder deren reaktive Derivate an ein oligomeres oder prepolymeres Grundgerüst gebunden enthalten.

**10.** Mischungen nach Anspruch 9, dadurch gekennzeichnet, daß die oligomeren oder prepolymeren Grundgerüste Homo-oder Copolymerisate von ethylenisch ungesättigten Monomeren sind.

**11.** Mischungen nach Anspruch 10, dadurch gekennzeichnet, daß sie poly(meth-)acrylierte Oligomaleinsäure, poly(meth-)acrylierte Polymaleinsäure, poly(meth-)acrylierte Poly(meth-)acrylsäure, poly(meth)-acrylierte Polycarbon-polyphosphonsäure, poly(meth-)acrylierte Polychlorophosphorsäure, poly(meth)-acryliertes Polysulfonat oder poly(meth-)acrylierte Polyborsäure enthalten.

**12.** Mischungen nach Anspruch 9, dadurch gekennzeichnet, daß die oligomeren oder polymeren Grundgerüste Polyester Polyamide, Polyether, Polysulfone, Polyphosphazene oder Polysaccharide sind.

**13.** Mischungen nach Anspruch 9 bis 12, dadurch gekennzeichnet, daß die Oligomeren ein Molekulargewicht von mindestens 500 aufweisen.

**14.** Mischungen nach Anspruch 9 bis 12, dadurch gekennzeichnet, daß die Prepolymeren ein Molekulargewicht von mindestens 1.500 aufweisen.

**15.** Mischungen nach Anspruch 9 bis 12, dadurch gekennzeichnet, daß die Prepolymeren ein Molekulargewicht von maximal 100.000 aufweisen.

**16.** Mischungen nach Anspruch 9 bis 12, dadurch gekennzeichnet, daß die Prepolymeren ein Molekulargewicht von maximal 20.000 aufweisen.

**17.** Mischungen nach Anspruch 1 bis 16, dadurch gekennzeichnet, daß die Monomeren, Oligomeren oder Prepolymeren außer den Säure- und polymerisierbaren Gruppen Aldehyd-, Epoxid-, Isocyanat- oder Halotriazingruppen enthalten.

**18.** Mischungen nach einem der Ansprüche 1 bis 17, dadurch gekennzeichnet, daß sie zusätzlich andere polymerisierbare ungesättigte Monomere und/oder
Oligomere und/oder Prepolymere enthalten, die keine Säuregruppen
oder deren reaktive, leicht hydrolysierbare Säurederivatgruppen aufweisen.

**19.** Mischungen nach einem der Ansprüche 1 bis 18, dadurch gekennzeichnet, daß sie zusätzlich andere Verbindungen enthalten, die Säuregruppen
oder deren reaktive, leicht hydrolysierbare Säurederivatgruppen aufweisen, aber keine Gruppen enthalten, die ungesättigt und polymerisierbar sind.

**20.** Mischungen nach einem der Ansprüche 1 bis 19, dadurch gekennzeichnet, daß die polymerisierbaren, säuregruppen-oder säurederivatgruppenhaltigen Verbindungen in einem Anteil von mindestens 5 % der

polymerisierbaren Verbindungen vorliegen.

**21.** Mischungen nach einem der Ansprüche 1 bis 19, dadurch gekennzeichnet, daß die polymerisierbaren, säuregruppen-oder säurederivatgruppenhaltigen Verbindungen in einem Anteil von 20 % bis 60 % der polymerisierbaren Verbindungen vorliegen.

**22.** Mischung nach einem Anspruch 1 bis 21, dadurch gekennzeichnet, daß die reaktiven Füllstoffe mit den Säuren, Salzen oder Säurederivaten reagierende, in feinteiliger Form vorliegende Metallverbindungen, Metallverbindungen enthaltende Gläser oder Keramiken oder Zeolithe, oxidierbare Metalle sowie Bornitrid sind oder Sinterprodukte von Mischungen dieser Metallverbindungen, dieselben enthaltenden Gläser oder Keramiken, Zeolithe und oxidierbare Metalle oder Sinterprodukte dieser Komponenten mit edleren Metallen darstellen.

**23.** Mischungen nach einem der Ansprüche 1 - 22, dadurch gekennzeichnet, daß die Metallverbindungen Metalloxide oder Metallhydroxide sind.

**24.** Mischungen nach einem der Ansprüche 1 - 22, dadurch gekennzeichnet, daß die Metallverbindungen enthaltenden Gläser oder Keramiken Silikatzementschmelzen oder ionenfreisetzende Gläser sind.

**25.** Mischungen nach einem der Ansprüche 1 - 22, dadurch gekennzeichnet daß feinteilige Pulver von Silber oder Silberlegierungen mit feinteiligen Pulvern von ionenfreisetzendem Glas oder Silikatzementschmelzen zusammen gesintert sind.

**26.** Mischungen nach einem der Ansprüche 1 - 25, dadurch gekennzeichnet, daß zusätzliche, im Sinne von Zementabbindereaktionen nicht reaktive, anorganische oder organische Füllstoffe zugemischt sind.

**27.** Mischungen nach einem der Ansprüche 1 - 26, dadurch gekennzeichnet, daß der Anteil der reaktiven Füllstoffe am Gesamtfüllstoffgehalt mindestens 5% beträgt.

**28.** Mischungen nach einem der Ansprüche 1 - 26, dadurch gekennzeichnet daß der Anteil der reaktiven Füllstoffe am Gesamtfüllstoffgehalt mindestens 30% beträgt.

**29.** Mischungen nach einem der Ansprüche 1 - 28, dadurch gekennzeichnet, daß der Anteil des Gesamtfüllstoffs zwischen 10% und 95% der Mischung beträgt.

**30.** Mischungen nach Anspruch 1 - 29, dadurch gekennzeichnet, daß das Härtungsmittel ein Polymerisationskatalysator oder -system ist.

**31.** Mischungen nach einem der Ansprüche 1 - 20, dadurch gekennzeichnet, daß das Polymerisationskatalysatorsystem lichtaktivierbar ist und aus einem Gemisch aus einem $\alpha$-Diketon und einem tertiären Amin und / oder einem tertiären Phosphin besteht.

**32.** Mischungen nach einem der Ansprüche 1 - 30, dadurch gekennzeichnet, daß das Polymerisationskatalysatorsystem aus 2 getrennten Komponenten besteht, wobei die eine Komponente ein organisches Peroxid und die andere Komponente ein tertiäres Amin, eine Schwefelverbindung, in der Schwefel in der Oxidationsstufe + 2 oder + 4 vorliegt, oder ein Gemisch der beiden ist, oder chelatbildende zweiwertige Metallionen enthält.

**33.** Mischungen nach Anspruch 32, dadurch gekennzeichnet, daß die Komponente eines 2-Komponenten Gemischs, die die Schwefelverbindung enthält, keine polymerisierbare säure- oder säuregruppenenthaltende Verbindungen, jedoch mindestens ein polymerisierbares Monomer mit Hydroxylgruppen enthält.

**34.** Vemwendung von Mischungen nach einem der Anspüche 1 - 33 als härtbare Mischungen zum Ausfüllen, Versiegeln und Kleben von oxidischen, mineralischen, glasartigen, keramischen, metallischen und biologischen Substraten.

**35.** Verwendung von Mischungen nach einem der Ansprüche 1 - 33 als haftvermittelnde Schicht zwischen oxidischem, mineralischem, glasartigem, keramischem, metallischem oder biologischem Substrat und

radikalisch polymerisierbaren Kunststoffmaterialien.

**36.** Verwendung von Mischungen nach Anspruch 1 - 33 zum Herstellen von ausgehärteten Formkörpern.

**37.** Verwendung von Mischungen nach Anspruch 1-36 zur Herstellung von Produkten oder Zubereitungen für dentale und medizinische Zwecke.

**Claims**

**1.** A polymerizable cement mixture containing

a) polymerizable unsaturated monomers and/or oligomers and/or prepolymers containing acid groups and/or reactive groups derived from acids,

b) finely divided, reactive fillers capable of reacting with these acids or acid derivatives, namely powders of phosphate cement (ZnO/MgO), silicate cements, ionomer cements or ion-exchange zeolites, and

c) hardeners,

characterized in that components a) and b) are chosen so that the acid groups or groups derived from acids according to a) are capable of reacting ionically with the finely divided reactive fillers according to b) to undergo a cement reaction.

**2.** A mixture according to Claim 1, characterized in that the polymerizable compounds contain at least two polymerizable groups and at least two acid groups or reactive groups derived from acids.

**3.** A mixture according to Claim 1, characterized in that the polymerizable compounds contain three or more polymerizable groups and three or more acid groups or reactive groups derived from acids.

**4.** A mixture according to one of Claims 1 to 3, characterized in that the polymerizable unsaturated compounds contain acrylic, methacrylic, vinyl or styryl groups or a mixture of these groups.

**5.** A mixture according to Claims 1 to 3, characterized in that the polymerizable unsaturated compounds contain acrylic or methacrylic groups.

**6.** A mixture according to one of Claims 1 to 5, characterized in that the acid groups are carboxylic acid radicals or salts thereof, phosphorus acid radicals of the formulae

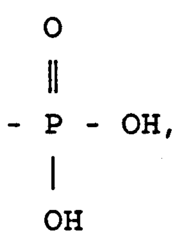 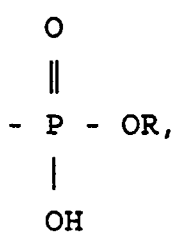 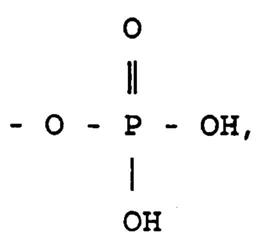 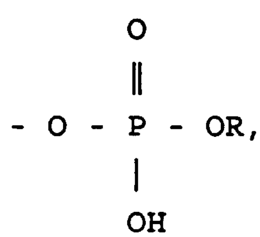

or salts thereof, wherein R is alkyl, aryl or vinyl, sulphur acid radicals of the formulae $-SO_2H$, $SO_3H$ or $-O-SO_3H$ or salts thereof, or boron acid radicals of the formulae

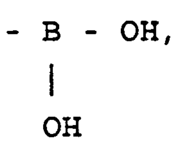 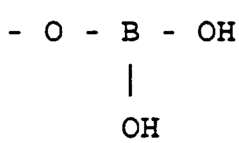 or 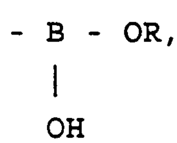 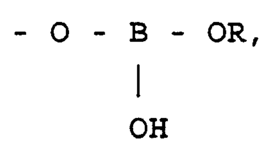

or salts thereof, wherein R is alkyl, aryl or vinyl.

7. A mixture according to one of Claims 1 to 6, characterized in that the reactive groups derived from acids in the form of acid halides or anhydrides.

8. A mixture according to Claims 1 to 7, characterized in that the unsaturated monomer is a halogenophosphoric acid ester of bis-GMA.

9. A mixture according to Claims 1 to 7, characterized in that the oligomers or prepolymers are compounds which contain the polymerizable unsaturated groups and the acid radicals, salts thereof or reactive derivatives thereof bonded to an oligomeric or prepolymeric skeleton.

10. A mixture according to Claim 9, characterized in that the oligomeric or prepolymeric skeletons are homo- or co-polymers of ethylenically unsaturated monomers.

11. A mixture according to Claim 10, characterized in that it contains poly(meth)acrylated oligomaleic acid, poly(meth)acrylated polymaleic acid, poly(meth)acrylated poly(meth)acrylic acid, poly(meth)acrylated polycarboxylic-polyphosphonic acid, poly(meth)acrylated polychlorophosphoric acid, poly(meth)-acrylated polysulphonate or poly(meth)acrylated polyboric acid.

12. A mixture according to Claim 9, characterized in that the oligomeric or polymeric skeletons are polyesters, polyamides, polyethers, polysulphones, polyphosphazenes or polysaccharides.

13. A mixture according to Claims 9 to 12, characterized in that the oligomers have a molecular weight of at least 500.

14. A mixture according to Claims 9 to 12, characterized in that the prepolymers have a molecular weight of at least 1500.

15. A mixture according to Claims 9 to 12, characterized in that the prepolymers have a molecular weight of at most 100,000.

16. A mixture according to Claims 9 to 12, characterized in that the prepolymers have a molecular weight of at most 20,000.

17. A mixture according to Claims 1 to 16, characterized in that the monomers, oligomers or prepolymers contain aldehyde, epoxide, isocyanate or halogenotriazine groups in addition to the acid groups and polymerizable groups.

18. A mixture according to one of Claims 1 to 17, characterized in that it additionally contains other polymerizable unsaturated monomers and/or oligomers and/or prepolymers which have no acid groups or easily hydrolyzable reactive groups derived from acids.

19. A mixture according to one of Claims 1 to 18, characterized in that it additionally contains other compounds which have acid groups or easily hydrolyzable reactive groups derived from acids, but contain no groups which are unsaturated and polymerizable.

20. A mixture according to one of Claims 1 to 19, characterized in that the polymerizable compounds containing acid groups or groups derived from acids are present in a proportion of at least 5% of the polymerizable compounds.

21. A mixture according to one of Claims 1 to 19, characterized in that the polymerizable compounds containing acid groups or groups derived from acids are present in a proportion of 20% to 60% of the polymerizable compounds.

22. A mixture according to one of Claims 1 to 21, characterized in that the reactive fillers are substances which are present in a finely divided form and react with the acids, salts or acid derivatives, said substances being metal compounds, glasses or ceramics containing metal compounds, or zeolites, oxidizable metals and boron nitride, or sintered products of mixtures of these metal compounds, glasses or ceramics containing the latter, zeolites and oxidizable metals, or sintered products of these

components with nobler metals.

23. A mixture according to one of Claims 1 - 22, characterized in that the metal compounds are metal oxides or metal hydroxides.

24. A mixture according to one of Claims 1 - 22, characterized in that the glasses or ceramics containing metal compounds are silicate cement melts or ion-releasing glasses.

25. A mixture according to one of Claims 1 - 22, characterized in that finely divided powders of silver or silver alloys are sintered together with finely divided powders of ion-releasing glass or silicate cement melts.

26. A mixture according to one of Claims 1 - 25, characterized in that additional inorganic or organic fillers which are unreactive in cement setting reactions are added.

27. A mixture according to one of Claims 1 - 26, characterized in that the proportion of reactive fillers in the total filler content is at least 5%.

28. A mixture according to one of Claims 1 - 26, characterized in that the proportion of reactive fillers in the total filler content is at least 30%.

29. A mixture according to one of Claims 1 - 28, characterized in that the proportion of total filler is from 10% to 95% of the mixture.

30. A mixture according to Claims 1 - 29, characterized in that the hardener is a polymerization catalyst or catalyst system.

31. A mixture according to one of Claims 1 - 20, characterized in that the polymerization catalyst system can be activated by light and consists of a mixture of an $\alpha$-diketone and a tertiary amine and/or a tertiary phosphine.

32. A mixture according to one of Claims 1 - 30, characterized in that the polymerization catalyst system consists of 2 separate components, one component containing an organic peroxide and the other component containing a tertiary amine, a sulphur compound in which sulphur is present in oxidation state +2 or +4, or a mixture of the two, or chelating divalent metal ions.

33. A mixture according to Claim 32, characterized in that the component of a 2-component mixture which contains the sulphur compound contains no polymerizable acid compounds or compounds containing acid groups, but does contain at least one polymerizable monomer having hydroxyl groups.

34. Use of mixtures according to one of Claims 1 - 33 as hardenable mixtures for filling, sealing and bonding oxidic, mineral, vitreous, ceramic, metallic and biological substrates.

35. Use of mixtures according to one of Claims 1 - 33 as a coupling layer between oxidic, mineral, vitreous, ceramic, metallic or biological substrate and plastic materials which can undergo radical polymerization.

36. Use of mixtures according to Claims 1 - 33 for the production of hardened moulded articles.

37. Use of mixtures according to Claims 1 - 36 for the production of products or preparations for dental and medical purposes.

**Revendications**

1. Mélanges de ciment polymérisables, contenant
   a) des monomères et/ou oligomères et/ou prépolymères insaturés polymérisables qui contiennent des groupes acides et/ou leurs groupes dérivés d'acides réactifs,
   b) des charges réactives finement divisées qui peuvent réagir avec ces acides ou dérivés d'acides à savoir de la poudre de ciments phosphatés (ZnO/MgO), de ciments silicatés, de ciment ionomérique

et de zéolite échangeuse d'ions, ainsi que

c) des durcissants,

caractérisés en ce que les composants a) et b) sont choisis de manière que les groupes acides ou les groupes dérivés d'acides selon a) peuvent conduire à une réaction de ciment de façon ionique avec les charges réactives finement divisées selon b).

**2.** Mélanges selon la revendication 1, caractérisés en ce que les composés polymérisables contiennent au moins deux groupes polymérisables et au moins deux groupes acides ou leurs groupes dérivés réactifs.

**3.** Mélanges selon la revendication 1, caractérisés en ce que les composés polymérisables contiennent trois groupes polymérisables ou plus et trois groupes acides, ou leurs groupes dérivés réactifs, ou plus.

**4.** Mélanges selon l'une des revendications 1 à 3, caractérisés en ce que les composés insaturés polymérisables contiennent des groupes acryliques, méthacryliques, vinyliques, styryliques ou un mélange de ces groupes.

**5.** Mélange selon les revendications 1 à 3, caractérisé en ce que les composés insaturés polymérisables contiennent des groupes acryliques ou méthacryliques.

**6.** Mélanges selon l'une des revendications 1 à 5, caractérisés en ce que les groupes acides sont des radicaux acide carboxylique ou leurs sels, des radicaux acide phosphorique de formules

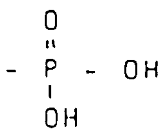 , 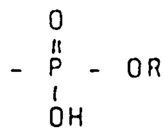 , 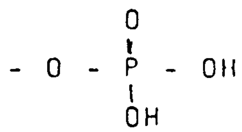 , 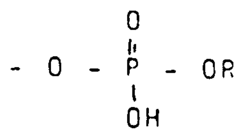

ou leurs sels, où R représente un alcoyle, aryle ou vinyle,
des radicaux acide sulfurique de formules

$-SO_2H$, $SO_3H$, $-O-SO_3H$

ou leurs sels ou
des radicaux acide borique de formules

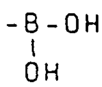 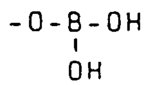 ou 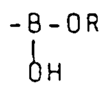 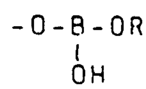

ou leurs sels, où R représente un alcoyle, aryle ou vinyle.

**7.** Mélanges selon l'une des revendications 1 à 6, caractérisés en ce que les groupes dérivés d'acides réactifs se présentent sous forme d'halogénures ou anhydrides d'acides.

**8.** Mélanges selon les revendications 1 à 7, caractérisés en ce que le monomère insaturé est un ester d'acide halophosphorique de Bis-GMA.

**9.** Mélanges selon les revendications 1 à 7, caractérisés en ce que les oligomères ou prépolymères sont des composés qui contiennent les groupes insaturés polymérisables et les radicaux acide, leurs sels ou leurs dérivés réactifs liés sur une ossature de base oligomère ou prépolymère.

**10.** Mélanges selon la revendication 9, caractérisés en ce que les ossatures de base oligomères ou prépolymères sont des homo- ou copolymères de monomères éthyléniquement insatures.

**11.** Mélanges selon la revendication 10, caractérisés en ce qu'ils contiennent de l'acide oligomaléique poly-(méth-)acrylé, de l'acide polymaléique poly(méth-) acrylède l'acide poly(méth-)acrylique poly(méth-) acrylé, de l'acide polycarboxylique-polyphosphonique poly(méth-)acrylé, de l'acide polychlorophosphorique poly(méth)acrylé, un polysulfonate poly(méth-)acrylé ou un acide polyborique poly(méth-)acrylé.

**12.** Mélanges selon la revendication 9, caractérisés en ce que les ossatures de base oligomères ou polymères sont des polyesters , polyamides, polyéthers, polysulfones, polyphosphazènes ou polysaccharides.

**13.** Mélanges selon les revendications 9 à 12, caractérisés en ce que les oligomères présentent un poids moléculaire d'au moins 500.

**14.** Mélanges selon les revendications 9 à 12, caractérisés en ce que les prépolymères présentent un poids moléculaire d'au moins 1500.

**15.** Mélanges selon les revendications 9 à 12, caractérisés en ce que les prépolymères présentent un poids moléculaire d'au maximum 100 000.

**16.** Mélanges selon les revendications 9 à 12, caractérisés en ce que les prépolymères présentent un poids moléculaire d'au maximum 20 000.

**17.** Mélanges selon les revendications 1 à 16, caractérisés en ce que les monomères, oligomères ou prépolymères contiennent, outre les groupes acides et polymérisables, des groupes aldéhyde, époxyde, isocyanate ou halotriazine.

**18.** Mélanges selon l'une des revendications 1 à 17, caractérisés en ce qu'ils contiennent en outre d'autres monomères et/ou oligomères et/ou prépolymères insaturés polymérisables qui ne présentent pas de groupes acides ou leurs groupes dérivés d'acides réactifs facilement hydrolysables.

**19.** Mélanges selon l'une des revendications 1 à 18, caractérisés en ce qu'ils contiennent en outre d'autres composés qui présentent des groupes acides ou leurs groupes dérivés d'acides réactifs facilement hydrolysables, mais ne contiennent pas de groupes qui sont insaturés et polymérisables.

**20.** Mélanges selon l'une des revendications 1 à 19, caractérisés en ce que les composés polymérisables contenant des groupes acides ou des groupes dérivés d'acides sont présents en une proportion d'au moins 5% des composés polymérisables.

**21.** Mélanges selon l'une des revendications 1 à 19, caractérisés en ce que les composés polymérisables contenant des groupes acides ou des groupes dérivés d'acides sont présents en une proportion de 20% à 60% des composés polymérisables.

**22.** Mélange selon l'une des revendications 1 à 21, caractérisé en ce que les charges réactives sont des composés métalliques, des verres contenant des composés métalliques ou des céramiques ou des zéolithes, des métaux oxydables ainsi que du nitrure de bore, présents sous forme finement divisée, réagissant avec les acides, sels ou dérivés d'acides, ou représentent des produits de frittage de mélanges de ces composés métalliques, de verres ou céramiques qui les contiennent, de zéolithes et de métaux oxydables, ou des produits de frittage de ces composants avec des métaux plus nobles.

**23.** Mélanges selon l'une des revendications 1-22, caractérisés en ce que les composés métalliques sont des oxydes métalliques ou des hydroxydes métalliques.

**24.** Mélanges selon l'une des revendications 1-22, caractérisés en ce que les verres ou céramiques contenant des composés métalliques sont des masses fondues de ciment silicaté ou des verres libérant des ions.

**25.** Mélanges selon l'une des revendications 1-22, caractérisés en ce que des poudres finement divisées d'argent ou d'alliages d'argent sont frittées avec des poudres finement divisées de verre libérant des ions ou de masses fondues de ciment silicaté.

26. Mélanges selon l'une des revendications 1-25, caractérisés en ce qu'on ajoute au mélange des charges supplémentaires inorganiques ou organiques non réactives au sens de réactions de durcissement de ciment.

27. Mélanges selon l'une des revendications 1-26, caractérisés en ce que la proportion des charges réactives par rapport à la teneur totale en charge s'élève à au moins 5%.

28. Mélanges selon l'une des revendications 1-26, caractérisés en ce que la proportion des charges réactives par rapport à la teneur totale en charges s'élève à au moins 30%.

29. Mélanges selon l'une des revendications 1-28, caractérisés en ce que la proportion des charges totales se situe entre 10% en 95% du mélange.

30. Mélanges selon les revendications 1-29, caractérisés en ce que l'agent durcissant est un catalyseur ou système de polymérisation.

31. Mélanges selon l'une des revendications 1-20, caractérisés en ce que le système de catalyseur de polymérisation est activable à la lumière et se compose d'un mélange d'une $\alpha$-dicétone et d'une amine tertiaire et/ou d'une phosphine tertiaire.

32. Mélanges selon l'une des revendications 1-30, caractérisés en ce que le système de catalyseur de polymérisation se compose de deux composants séparés, où un composant est un peroxyde organique et l'autre composant est une amine tertiaire, un composé de soufre dans lequel le soufre se présente au degré d'oxydation +2 ou +4, ou un mélange des deux, ou contient des ions métalliques bivalents formateurs de chélates.

33. Mélanges selon la revendication 32, caractérisés en ce que le composant d'un mélange à deux composants qui contient le composé de soufre ne contient pas de composé acides ou contenant des groupes acides polymérisables, mais contient au moins un monomère polymérisable ayant des groupes hydroxyle.

34. Application de mélanges selon l'une des revendications 1-33 comme mélanges durcissables pour le remplissage, l'obturation et l'adhérence de substances oxydiques, minérales, vitreuses, céramiques, métalliques et biologiques.

35. Application de mélanges selon l'une des revendications 1-33 comme couche adhésive entre un substrat oxydique , minéral , vitreux , céramique, métallique ou biologique et des matières synthétiques capables de subir une polymérisation radicalaire.

36. Application de mélanges selon les revendications 1-33 à la préparation de pièces moulées durcies.

37. Application de mélanges selon les revendications 1-36 à la préparation de produits ou de préparations dans des buts dentaires et médicaux.